# EUROPEAN PATENT APPLICATION

(11) **EP 3 501 511 A1**
(43) Date of publication of application: **26.06.2019**
(21) Application number: 17382891.4
(22) Date of filing: 22.12.2017
(51) Int. Cl.: A61K 31/00, A61K 31/05, A61K 31/357, A61K 31/44, A61K 31/7088, C12N 15/113, C07K 16/18, A61P 35/04

(54) **MEANS FOR PREVENTING AND TREATING BRAIN METASTASIS**

(71) Applicant: Fundación Centro Nacional De Investigaciones Oncológicas Carlos III, 28029 Madrid (ES); Institut Català d'Oncologia (ICO), 08908 L'Hospitalet de Llobregat (ES); Fundacio Institut d'Investigació Biomèdica de Girona Dr. Josep Trueta, 17190 Salt (Girona) (ES)
(72) Inventor: VALIENTE CORTÉS, Manuel, 28010 Madrid (ES); PRIEGO BENDECK, Neibla, 28029 Madrid (ES); BOSCH BARRERA, Joaquim, 17190 Salt (ES)
(74) Representative: Elzaburu S.L.P.

(57) **Abstract**

The present invention provides means for preventing and/or treating brain metastasis. The present invention relates to a novel method of preventing and/or treating brain metastasis by acting specifically on astrocytes for inhibiting the expression or the activation of STAT3 in these cells.

SEQUENCE LISTING

<110>  Fundación Centro Nacional de Investigaciones Oncológicas
       Carlos III (CNIO)
       Institut Català d'Oncologia
       Fundació Institut d'Investigació Biomèdica de Girona Dr.
       Josep Trueta

<120>  MEANS FOR PREVENTING AND TREATING BRAIN METASTASIS

<130>  EP-1722

<160>  31

<170>  PatentIn version 3.5

<210>  1
<211>  19
<212>  DNA
<213>  Homo sapiens

<400>  1
aagtttctaa acagctcca                                                    19


<210>  2
<211>  20
<212>  DNA
<213>  Homo sapiens

<400>  2
ctgagctggc agttctcctc                                                   20


<210>  3
<211>  20
<212>  DNA
<213>  Homo sapiens

<400>  3
gaaggtgcct ggaggcttag                                                   20


<210>  4
<211>  22
<212>  DNA
<213>  Mus musculus

<400>  4
cagaaagtgt cctacaaggg cg                                                22


<210>  5
<211>  23
<212>  DNA
<213>  Mus musculus

<400>  5
cgttgttaga ctcctccatg ttc                                               23


<210>  6
<211>  20

<212>  DNA
<213>  Mus musculus

<400>  6
acgttaagct agccctggac                                                    20


<210>  7
<211>  21
<212>  DNA
<213>  Mus musculus

<400>  7
ggtgagcctg tattgggaca a                                                  21


<210>  8
<211>  20
<212>  DNA
<213>  Mus musculus

<400>  8
tttgagctga gcgagatcgg                                                    20


<210>  9
<211>  20
<212>  DNA
<213>  Mus musculus

<400>  9
agaggtctca gctccacact                                                    20


<210>  10
<211>  18
<212>  DNA
<213>  Mus musculus

<400>  10
gtgccagaac gcacactg                                                      18


<210>  11
<211>  18
<212>  DNA
<213>  Mus musculus

<400>  11
ccccggttac actccaaa                                                      18


<210>  12
<211>  22
<212>  DNA
<213>  Mus musculus

<400>  12
gagacacacc agagcagata cc                                                 22


<210>  13
<211>  21

<212>  DNA
<213>  Mus musculus

<400>  13
tggtctcgtt gatttctggg g                                                21


<210>  14
<211>  20
<212>  DNA
<213>  Mus musculus

<400>  14
ttcacagcct gctgtcactt                                                  20


<210>  15
<211>  20
<212>  DNA
<213>  Mus musculus

<400>  15
ctctccccct gaagttgctg                                                  20


<210>  16
<211>  20
<212>  DNA
<213>  Mus musculus

<400>  16
gaacttgatc cctgccccat                                                  20


<210>  17
<211>  21
<212>  DNA
<213>  Mus musculus

<400>  17
ttctgatcca gtagcgacag c                                                21


<210>  18
<211>  30
<212>  DNA
<213>  Mus musculus

<400>  18
cttcccaatg cgttcgaaga agatttttgg                                       30


<210>  19
<211>  30
<212>  DNA
<213>  Mus musculus

<400>  19
aacacttaag aaacatactg ggctcctccg                                       30


<210>  20
<211>  26

<212>  DNA
<213>  Mus musculus

<400>  20
gccgaattgt gtcttggtgg atgaca                                          26


<210>  21
<211>  25
<212>  DNA
<213>  Mus musculus

<400>  21
cctggtggaa tgcactgcag aagga                                           25


<210>  22
<211>  21
<212>  DNA
<213>  Mus musculus

<400>  22
cctggccctc aagtacacct t                                               21


<210>  23
<211>  20
<212>  DNA
<213>  Mus musculus

<400>  23
tccgtacgac gcatttctag                                                 20


<210>  24
<211>  20
<212>  DNA
<213>  Mus musculus

<400>  24
ctgctgaccc ccactgatac                                                 20


<210>  25
<211>  20
<212>  DNA
<213>  Mus musculus

<400>  25
gtgagcgctg aatcgaaagc                                                 20


<210>  26
<211>  20
<212>  DNA
<213>  Mus musculus

<400>  26
ctttgtaccg tcctccggtc                                                 20


<210>  27
<211>  20

<212>  DNA
<213>  Mus musculus

<400>  27
cgttcgtgcc gctaaaagtc                                                    20


<210>  28
<211>  20
<212>  DNA
<213>  Homo sapiens

<400>  28
agatgagtat gcctgccgtg                                                    20


<210>  29
<211>  20
<212>  DNA
<213>  Homo sapiens

<400>  29
tcatccaatc caaatgcggc                                                    20


<210>  30
<211>  21
<212>  DNA
<213>  Mus musculus

<400>  30
ggcaccacac cttctacaat g                                                  21


<210>  31
<211>  20
<212>  DNA
<213>  Mus musculus

<400>  31
gtggtggtga agctgtagcc                                                    20

## Description

### FIELD OF INVENTION

The present invention relates to brain metastasis and means for preventing and treating it. More specifically, the invention relates to a novel method of preventing and/or treating brain metastasis by acting specifically on astrocytes for inhibiting the expression or the activation of STAT3 in these cells.

### BACKGROUND OF THE INVENTION

Brain metastases are the most common intracranial neoplasm in adults. Although the exact incidence is unknown, it has been estimated to be as high as 200,000 cases per year in the USA alone. An alarming fact is that approximately 20-40% of all cancers develop brain metastases. This accounts for 98,000 to 170,000 new brain metastasis cases each year in the USA alone. Approximately 30% of all cancer patients have detectable metastases at the moment of clinical diagnosis and a further 30% of patients have occult metastases. Approximately 20% of all brain metastases are single tumors (El-Habashy S.L. et al. Pharm Pat Anal. 3 279-296 (2014)).

The brain microenvironment imposes a particularly intense selective pressure on metastasis initiating cells, but successful metastasis bypass this control through mechanisms that are poorly understood. Reactive astrocytes are key components of this microenvironment, as they have been observed to confine brain metastasis without infiltrating the lesion.

Astrocytes respond to injury by activating a program that induces morphological and transcriptomic changes and brings them into a reactive state (Sofroniew, M. V. Trends Neurosci 32, 638-647 (2009)). Reactive astrocytes, characterized by high levels of GFAP, have been reported to improve or worsen the progression of various diseases (Sofroniew, M. V. Trends Neurosci 32, 638-647 (2009)), including brain metastasis (Valiente, M. et al. Cell 156, 1002-1016 (2014)), Chen, Q. et al. Nature 533, 493-498 (2016)) suggesting that this population is heterogeneous.

The STAT family of transcription factors (signal transducers and activators of transcription) transduce signals from cytokine receptors to the nucleus, where STAT dimers bind to DNA and regulate transcription. STAT3 is the most ubiquitous of the STATs, being activated by a wide variety of cytokines and growth factors. STAT3 has many roles in physiological processes such as inflammatory signalling, aerobic glycolysis and immune suppression, and was also the first family member shown to be aberrantly activated in a wide range of both solid and liquid tumours. STAT3 promotes tumorigenesis by regulating the expression of various target genes, including cell-cycle regulators, angiogenic factors and anti-apoptosis genes. Direct silencing or inhibition of STAT3 diminishes tumour growth and survival in both animal and human studies (Wake, M.S. and Watson C.J. FEBS Journal 282 2600-2611 (2015)).

Activation of Stat3 signaling in reactive astrocytes has been reported in central nervous system disorders including acute traumatic injury, ischemia, neurodegenerative and autoimmune diseases (Okada, S. et al. Nat Med 12, 829-834 (2006); Wanner, I. B. et al. J Neurosci 33, 12870-12886 (2013); LeComte, M. D., et al. Proc Natl Acad Sci U S A 112, 8726-8731 (2015); Ben Haim, L. et al. J Neurosci 35, 2817-2829 (2015); Jee, Y. et al. J Neuroimmunol 114, 40-47 (2001)).

Stat3 has been previously shown to be important in brain metastasis (Lee, H.-T. et al. Oncotarget 6, 10016-10029 (2015); Kong, L.-Y. et al. Clin Cancer Res 14, 5759-5768 (2008); Singh, M. et al. Oncotarget 6, 27461-27477 (2015); Bosch-Barrera, J. et al. Oncotarget 7, 32006-32014 (2016)). WO2016201188A1 relates to the use of Mitohonokiol for treating cancer through STAT3 inhibition. Although other components of the microenvironment have been proposed to mediate the pro-metastatic role of Stat3, reactive astrocytes (RA) have not been considered.

The treatment of brain metastasis remains challenging. Despite multimodal treatment approaches, the prognosis of brain metastases remains poor. Untreated patients with brain metastasis have a median survival of about 1 month, with almost all patients dying from neurological causes.

Thus, there is an unmet clinical need for effective means for preventing and/or treating brain metastasis.

### SUMMARY OF THE INVENTION

The present invention relates to a method for the prevention and/or treatment of brain metastasis comprising the administration of a therapeutically effective amount of an inhibitor of the expression or the activation of STAT3 in a patient in need thereof, wherein said inhibitor, in the brain, targets astrocytes and/or GFAP+ cells specifically.

### DESCRIPTION OF THE INVENTION

The present invention provides new means for the prevention and/or treatment of brain metastasis. The inventors of the present invention have surprisingly found that inhibiting STAT3 specifically in the reactive astrocytes that surround the brain metastasis reduces brain metastasis burden. The inventors have shown that blocking the expression of Stat3 in GFAP expressing cells prevents brain metastasis and reduces the size of the brain metastases that are already established.

Therefore, in a first aspect, the present invention relates to an inhibitor of the expression or the activation of STAT3 for use in the treatment and/or prevention of brain metastasis, wherein said inhibitor acts exclusively in astrocytes and/or GFAP+ cells or targets astrocytes and/or GFAP+ cells specifically.

The expression "inhibitor of the expression or the activation of STAT3" as used herein means that the inhibitor either prevents the expression or the activation of STAT3 or of other members of STAT3 pathway or combinations thereof. Preferably, the inhibitor prevents the expression or activation of the transcription factor STAT3. STAT3 is a transcription factor that is activated by phosphorylation of tyrosine 705 or serine 727. Then, the transcription factor dimerizes via the SH2 domain and translocate to the nucleus to bind to specific DNA elements to regulate gene expression. Therefore, preventing the expression of STAT3 inhibits STAT3 activity and also preventing STAT3 phosphorylation also inhibits STAT3 activity. In a preferred embodiment, the STAT3 inhibitor is specific and does nor inhibit other STATs such as STAT1 or STAT5.

The term "effective amount" or "therapeutically effective amount" as used herein refers to an amount that results in an improvement or remediation of the symptoms of the disease or condition.

In a preferred embodiment, the term "brain metastasis" as used herein refers to macrometastasis and does not include micrometastasis. Brain macrometastasis are large enough to be discernable by clinical radiological means, such as magnetic resonance imaging, computerized tomography, or positron emission tomography and are symptomatic, while brain micrometastasis are too small to be visualized by radiological means and are asymptomatic. The symptoms of brain metastasis are for example headache, vomiting or nausea, mental changes, such as increasing memory problems, confusion, changes in behaviour and personality, problems with balance and coordination, loss of bladder or bowel control (incontinence), problems with speech, problems with swallowing, seizures, weakness or numbness in parts of the body, such as the face, arms or legs and dizziness.

The terms "prevention" or "preventing" as used herein mean that the inhibitor can be administered to a subject or patient who does not show any brain metastasis symptoms but who would normally be expected to develop brain metastasis or be at increased risk for having brain metastasis. The inventors have found that STAT3 inhibition in reactive astrocytes prevents the development of macrometastasis and therefore increases overall survival. Particularly, patients with HER2 breast cancer, triple negative breast cancer, melanoma, small cell lung cancer (SCLC) or stage III non-squamous non-small cell lung cancer (NSCLC) possess the highest risk of developing brain metastasis (Mamon, H.J. et al. (2005) J Clin Oncol 23, 1530-1537; Steeg, P.S. et al. (2011) Nat Rev Cancer 11, 352-363).

The expressions "targets astrocytes specifically" and "acts exclusively in astrocytes" as used herein means that the inhibition of the expression or the activation of STAT3 happens mainly in astrocytes, preferably in reactive astrocytes, which show increased levels of GFAP. This means that the inhibitor is not a simple STAT3 inhibitor that acts on any cell, including cancer cells, but it acts preferentially on astrocytes and/or GFAP+ cells, it is targeted to act on astrocytes and/or GFAP+ cells. This means that other cells such as ependymal cells which may also be GFAP+ cells may also be subject to the inhibition of the expression or the activation of STAT3. In a preferred embodiment, the term "targets astrocytes and/or GFAP+ cells specifically" means that the percentage of pSTAT3+/GFAP+ cells that can be detected before administering the inhibitor is reduced in at least 2 fold after administering the inhibitor while in cancer cells there is no such a reduction. In a preferred embodiment, the inhibitor achieves a percentage of inhibition of STAT3 activation in GFAP+ cells that is at least 2 times bigger than in cancer cells. The quantification of pSTAT3+/GFAP+ cells can be performed by known methods such as immunocytochemistry of brain tissue of animal models of brain metastasis or of organotypic slice cultures (both disclosed in the examples). pSTAT3 means phosphorylated STAT3, preferably STAT3 phosphorylated at tyrosine 705. In brain models of brain metastasis, cancer cells can be easily quantified since they express green fluorescent protein (Gfp). In human tissue, cancer cells can be quantified by methods known by the skilled person, for example after immunostaining TTF1 (thyroid transcription factor 1) for lung cancer brain metastasis, MelanA (melanoma antigen recognized by T cells 1 or MART-1) and HMB 45 (Human Melanoma Black) for melanoma brain metastasis and Mammaglobin and BRST-2 (also known as GCDFP-15 for Gross Cystic Disease Fluid Protein 15) for breast cancer brain metastasis.

Astrocytes are glial cells characterised by the expression of glial fibrillary acidic protein (GFAP). Importantly, GFAP expression is increased in reactive astrocytes. Other proteins predominantly expressed in astrocytes are the glutamate aspartate transporter or the S100 calcium-binding protein B.

In a preferred embodiment of any one of the aspects of the present invention, the inhibitor crosses the blood-brain barrier.

In a preferred embodiment of the first aspect, the inhibitor comprises an inhibitory anti-STAT3 antibody or an antigen-binding fragment thereof, a decoy peptide, a nucleotide sequence or a STAT3 inhibitor compound. An inhibitory anti-STAT3 antibody or an antigen-binding fragment thereof can be any antibody or fragment thereof which specifically binds STAT3 and prevents its phosphorylation or its dimerization. Also, it can be an antibody or fragment thereof which binds to another member of the STAT3 pathway and ultimately inhibits STAT3 expression or activation. A nucleotide sequence that codifies for a decoy peptide can also be used as inhibitor according to the present invention. The STAT3 inhibitor compound can be a natural compound or a synthetic compound, what is commonly known as a small molecule or either natural or synthetic origin. Besides small peptides and oligonucleotides, numerous small molecules have been identified as blockers of STAT3 activation, including synthetic molecules (e.g., AG 490, decoy peptides, and oligonucleotides) and plant polyphenols. Examples of small molecules are the STAT3 inhibitors disclosed in WO2010117438A2, WO2011163424A2, WO2012018868A1 or in WO2014153495A2.

In a preferred embodiment of the first aspect of the present invention, the inhibitor comprises a nucleotide sequence which is or codifies for a gRNA (guide RNA or single guide RNA), a siRNA (small interfering RNA), a microRNA or a shRNA (short hairpin or small hairpin RNA). In a preferred embodiment of the fist aspect of the present invention, the inhibitor comprises the shRNA for human STAT3 of mature antisense sequence AAGTTTCTAAACAGCTCCA (SEQ ID NO: 1).

In a preferred embodiment of the first aspect of the present invention, the inhibitor is a nucleotide sequence expressed under the glial fibrillary acidic protein promoter, the glutamate aspartate transporter promoter or the S100 calcium-binding protein B promoter.

In a preferred embodiment of the fist aspect of the present invention, the inhibitor is delivered by a viral vector with lyssavirus rabies virus and/or Mokola virus type envelopes. This vector targets astrocytes specifically as disclosed in US 7749973 B2. Also in a preferred embodiment of the fist aspect of the present invention, the inhibitor is delivered by a adeno-associated viral vector, preferably of serotypes 2 or 5. These vectors also target astrocytes preferentially. In another preferred embodiment of the first aspect of the present invention, the inhibitor is delivered in nanoparticles.

In a preferred embodiment of the fist aspect of the present invention, the STAT3 inhibitor compound comprises silibinin, WP1066, WP1220 or honokiol. For example, any one of these STAT3 inhibitors or their derivatives can be vehiculized in nanoparticles or bound to specific antibodies which target astrocytes and/or GFAP + cells for use in the prevention and/or treatment of brain metastasis as disclosed in the present invention.

In a preferred embodiment of the fist aspect of the present invention, the STAT3 inhibitor compound is bound to a compound that is preferentially consumed by astrocytes or to an antibody against an antigen preferentially present in astrocytes or an antigen-binding fragment thereof. Preferably, said antigen preferentially present in astrocytes is CD54 or CD274.

In a preferred embodiment of the fist aspect of the present invention, the brain metastasis is a lung cancer, breast cancer, colon cancer, melanoma, renal carcinoma, head and neck cancer, prostate cancer, lymphoma, leukemia, hepatocellular carcinoma, bladder cancer, pancreatic cancer, gastric cancer, esophageal cancer, ovarian cancer or endometrial carcinoma brain metastasis. Preferably, the brain metastasis is a lung cancer, breast cancer, colon cancer, melanoma or renal carcinoma brain metastasis. More preferably, the brain metastasis is a lung cancer, breast cancer or melanoma brain metastasis.

In another preferred embodiment of the first aspect of the present invention, the method further comprises administering at least one cytotoxic chemotherapeutic agent, immunotherapy, targeted therapy, surgery, radiotherapy, stereotactic radiosurgery or combinations thereof. In a preferred embodiment, the therapy that is administered in addition to the inhibitor of the expression or the activation of STAT3 of the present invention is selected depending on the primary tumor.

Immunotherapy as used herein are treatments directed towards inducing or enhancing the immune response to destroy tumors by means of immunomodulators.

Targeted cancer therapies are drugs or other substances that block the growth and spread of cancer by interfering with specific molecules ("molecular targets") that are involved in the growth, progression, and spread of cancer. Many different targeted therapies have been approved for use in cancer treatment. These therapies include hormone therapies, signal transduction inhibitors, gene expression modulators, apoptosis inducers, angiogenesis inhibitors, immunotherapies, and toxin delivery molecules.

In a preferred embodiment of the present invention, the patient is human.

A second aspect of the present invention relates to an inhibitor of the expression or the activation of STAT3 wherein said inhibitor preferably crosses the blood-brain barrier, wherein said inhibitor, in the brain, targets astrocytes specifically, and wherein the inhibitor comprises an inhibitory anti-STAT3 antibody or an antigen-binding fragment thereof, a decoy peptide, a nucleotide sequence or a STAT3 inhibitor compound.

In a preferred embodiment of the second aspect of the invention, the inhibitor is a nucleotide sequence which is or codifies for a gRNA, a siRNA, a microRNA or a shRNA. Preferably, the inhibitor is a nucleotide sequence expressed under the glial fibrillary acidic protein promoter, the glutamate aspartate transporter promoter or the S100 calcium-binding protein B promoter.

In another preferred embodiment of the second aspect, the inhibitor is delivered by a viral vector with lyssavirus rabies virus and/or Mokola virus type envelopes or by adeno-associated viral vector, preferably of serotypes 2 or 5 or is delivered in nanoparticles.

In a preferred embodiment of the second aspect of the invention, the inhibitor comprises a compound selected from silibinin, WP1066, WP1220 or honokiol.

In a preferred embodiment of the second aspect of the invention, the STAT3 inhibitor compound is bound to a compound that is preferentially consumed by astrocytes or to an antibody against an antigen preferentially present in astrocytes or an antigen-binding fragment thereof. Preferably, the antigen preferentially present in astrocytes is CD54 or CD274.

A third aspect of the present invention relates to a composition comprising the inhibitor of the second aspect and at least one pharmaceutically acceptable excipient.

A fourth aspect of the present invention relates to the composition of the first aspect for use in the prevention and/or treatment of central nervous system disorders selected from brain metastasis, primary brain tumors, acute traumatic injury, ischemia, neurodegenerative and autoimmune diseases, preferably brain metastasis, more preferably lung cancer, breast cancer or melanoma brain metastasis.

### DESCRIPTION OF THE DRAWINGS

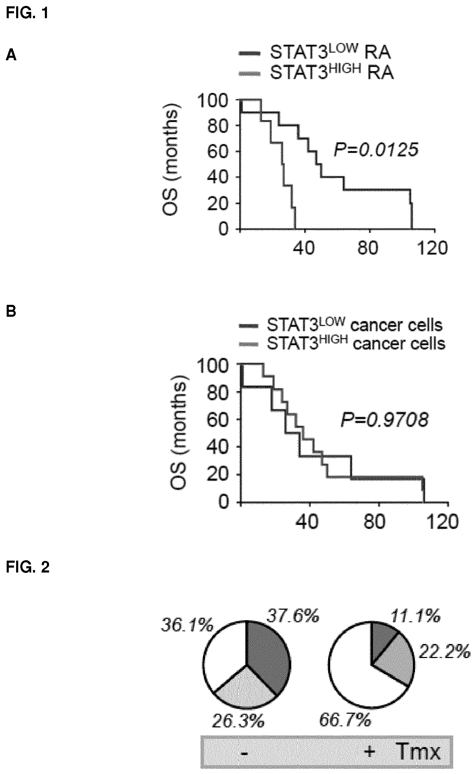

**Figure 1****.** Stat3 labels a subpopulation of reactive astrocytes associated with established brain metastasis independently of the origin of the primary tumor. A. Overall survival from diagnosis of the primary tumor to death was available for 17/ 29 patients. A survival curve was generated grouping patients with high (Score 3 and 2) and low (Score 1 and 0) pSTAT3 in the microenvironment. P values were obtained with log rank (Mantel-Cox) test. B. Same patients as in Fig.1A were used to generate a survival curve according to the same criteria but scoring pSTAT3 in cancer cells. P values obtained with log rank (Mantel-Cox) test.
**Figure 2****.** Distribution of B16/F10-BrM metastases according to the associated pStat3 signal in the surrounding microenvironment (n=28 control metastases, 7 tamoxifen treated metastases) comparing Tamoxifen treated and untreated cKO-Stat3 mice. Representative images of Gfp+ B16/F10-BrM metastases scored according to the amount of pStat3 (Tyr705) signal in the surrounding microenvironment. In Tamoxifen untreated cKO-Stat3 mice there was 36.1 % of Score 0 (no pStat3 signal), 26.3 % of Score 1 (low pStat3 signal) and 37.6 % of Score 2 (high pStat3 signal), while in Tamoxifen treated cKO-Stat3 mice there was 66.7 % of Score 0 (no pStat3 signal), 22.2 % of Score 1 (low pStat3 signal) and 11.1 % of Score 2 (high pStat3 signal).

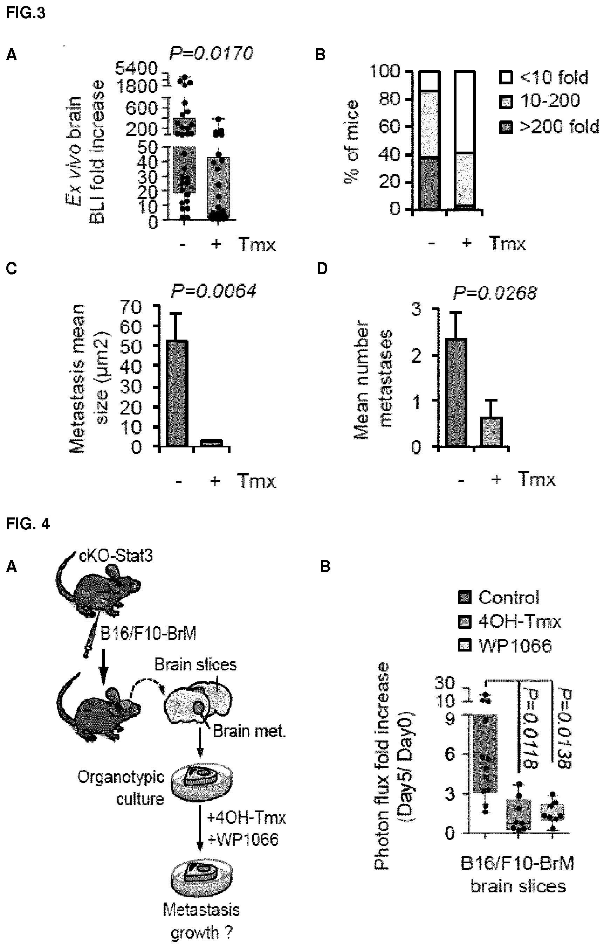

**Figure 3****.** Genetic targeting of Stat3 in reactive astrocytes impairs the viability of brain metastasis. A. Quantification of bioluminescence imaging (BLI). Every dot represents a different animal. Values were obtained from normalizing ex vivo brain signal to in vivo head signal 3 days after IC injection when treatment was initiated. Error bars, S.E.M. (n=29 mice per experimental condition, 5 independent experiments). P value is calculated using two-tailed t test. B. Quantification showing the percentage of mice in each category according to the BLI fold increase values. C. Quantification of the size of metastatic lesions in the cKO-Stat3 in Tamoxifen treated or untreated mice. Error bars, S.E.M. (n=5 and 6 metastases respectively, 2 independent experiments). P value is calculated using two-tailed t test. D. Quantification of the mean number of Gfp+ B16/F10-BrM brain metastases found in cKO-Stat3 Tamoxifen treated or untreated mice. Error bars, S.E.M. (n=11 brains per experimental condition). P value is calculated using two-tailed t test.

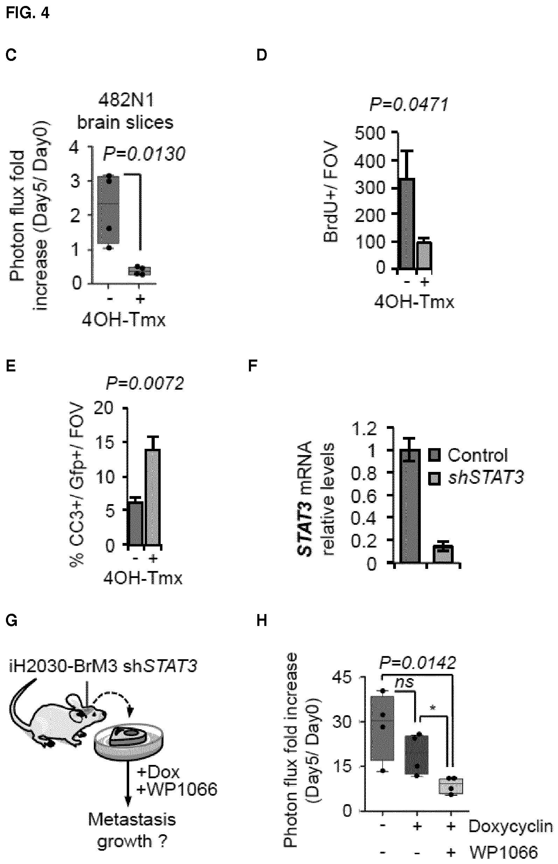

**Figure 4****.** A. Schema of experimental design. 4OH-Tamoxifen (4OH-Tmx) or WP1066 were added to cKO-Stat3 brain slices containing established B16/F10-BrM (melanoma origin) metastases. B. Quantification of the bioluminescence signal emitted by B16/F10-BrM cells in each brain slice normalized by the initial value obtained at day 0 (before the addition of either 4OH-Tmx or WP1066). Every dot represents a different organotypic culture. Error bars, S.E.M. (n= 12 control and 8 4OH-Tamoxifen and 8 WP1066 treated organotypic cultures, 2 independent experiments). P value is calculated using two-tailed t test. C. Quantification of the bioluminescence signal emitted by 482N1 (lung cancer origin) established metastases in CKO-Stat3 mice comparing 4OH-Tmx (1µM) treated and untreated cultures. Every dot represents an individual organotypic culture. Error bars, S.E.M. (n=4 organotypic cultures per experimental condition). P value is calculated using two-tailed t test. D. Quantification of the percentage of BrdU positive cancer cells in cKO-Stat3 organotypic cultures treated with 4OH-Tmx. Error bars, S.E.M. (n=8 field of view from 3 metastases per experimental condition). P value is calculated using two-tailed t test. E. Quantification of the percentage of cleaved Caspase 3 positive cancer cells in cKO-Stat3 organotypic cultures treated with 4OHTmx. Error bars, S.E.M. (n=4 field of view from 2 metastases per experimental condition). P value is calculated using two-tailed t test. F. qRT-PCR against human STAT3 was performed on brain slices shown in Fig. 4H after being cultured for five days in the presence or absence of Doxycycline. G. Schema of experimental design. Doxycycline (1 µg/ml) or WP1066 (10µM) were added to slices containing established H2030-BrM metastases previously transduced with a Doxycycline inducible shRNA against human STAT3 (iH2030-BrM shSTAT3). BLI was obtained before the addition of either Doxycycline or WP1066 to the media (Day 0). Five days later BLI was performed to analyze metastasis growth ex vivo by normalizing BLI values to day 0, before the addition of either Doxycycline or WP1066 to the media. H. Quantification of the bioluminescence signal emitted by iH2030-BrM shSTAT3 cells in each brain slice normalized by the initial value obtained at day 0, before the addition of any treatment. Every dot represents a different organotypic culture. Error bars, S.E.M. (n=4 organotypic cultures per experimental condition). P value is calculated using two-tailed t test.

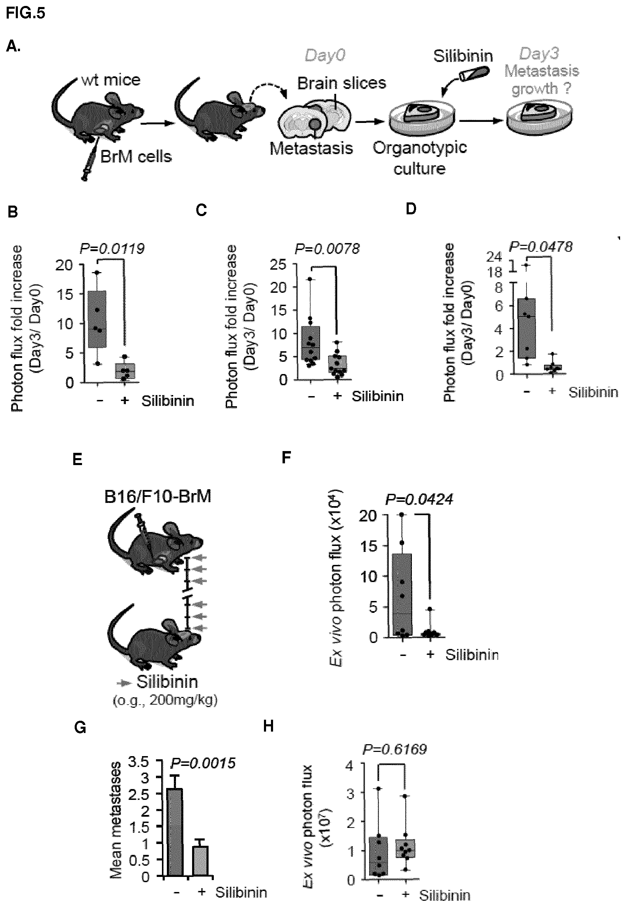

**Figure 5****.** Pharmacological targeting of Stat3+ reactive astrocytes impairs the viability of brain metastasis in mice and human. A. Schema of experimental design. Wt: wild type. B,C. Quantification of bioluminiscence in brain organotypic cultures with B16/F10-BrM (B), H2030-BrM (C) and 482N1 (D) established metastases that were grown *ex vivo* during three days in the presence or absence of silibinin (100 µM). Error bars, S.E.M. (n=5-12 organotypic cultures per experimental condition). P value is calculated using two-tailed t test. E. Schema of experimental design. Silibinin was administered by oral gavage (o.g.) daily during two weeks. F. Quantification of the bioluminescence in ex vivo images of the brain in control and silibinin treated animals at the endpoint of the experiment. Error bars, S.E.M. (Control: n=8 brains; silibinin: n=9 brains). P value is calculated using two-tailed t test. G. Quantification of the number of metastases found in silibinin treated and untreated brains. Error bars, S.E.M. (Control: n=8 brains; silibinin: n=9 brains). P value is calculated using two-tailed t test. H. Quantification of the bioluminescence in *ex vivo* images of the thorax and abdomen of mice of figure 5F. Error bars, S.E.M. (Control: n=8 mice; silibinin: n=9 mice). P value is calculated using two-tailed t test.

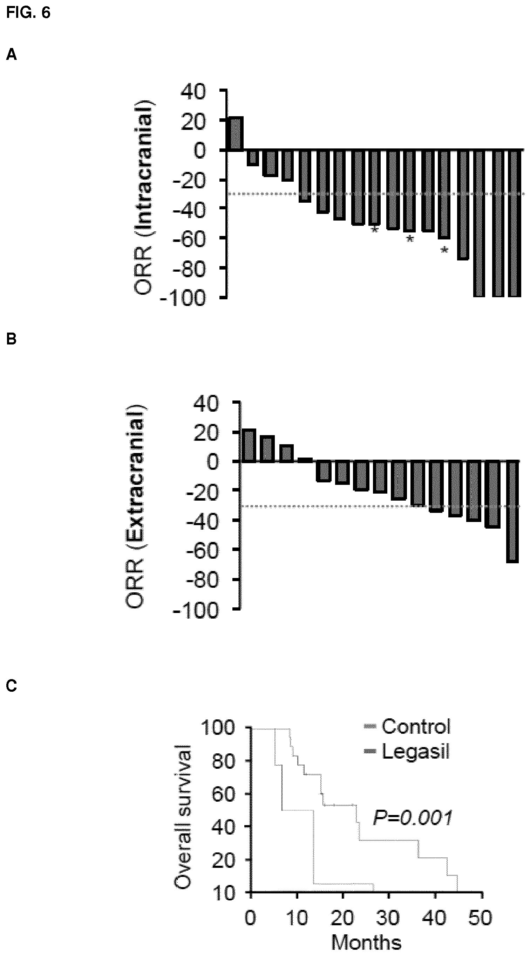

**Figure 6****.** A. Intracranial overall response rate (ORR) waterfall of 17 patients with lung cancer brain metastases who received Legasil®. Dotted grey line indicates 30% reduction of metastases. One patient was excluded from the waterfall plot because death ocurred before the first control brain MRI could be performed. Asterisks indicate patients who receive Legasil® under palliative care. B. Overall response rate (ORR) waterfall plot from extracranial disease (primary tumors and extracranial metastases) in 15 out of the 18 patients with lung cancer metastatic to the brain that received Legasil®. 3 excluded patients did not have extracranial disease (2) or target (measurable) lesion according to RECIST v1.1 criteria. Dotted grey line indicates 30% reduction. C. Overall survival (OS) from diagnosis of brain metastasis of 18 patients who received Legasil® compared to OS of the same patients calculated by Lung-molGPA tool (brain GPA Index33) Kaplan-Meier survival curves were generated. P value is calculated by log-rank test. Control line is the one on the left while Legasil® line is the one on the right. Kaplan-Meier survival curves were generated. P value is calculated by log-rank test. Control line is the one on the left while Legasil® line is the one on the right.

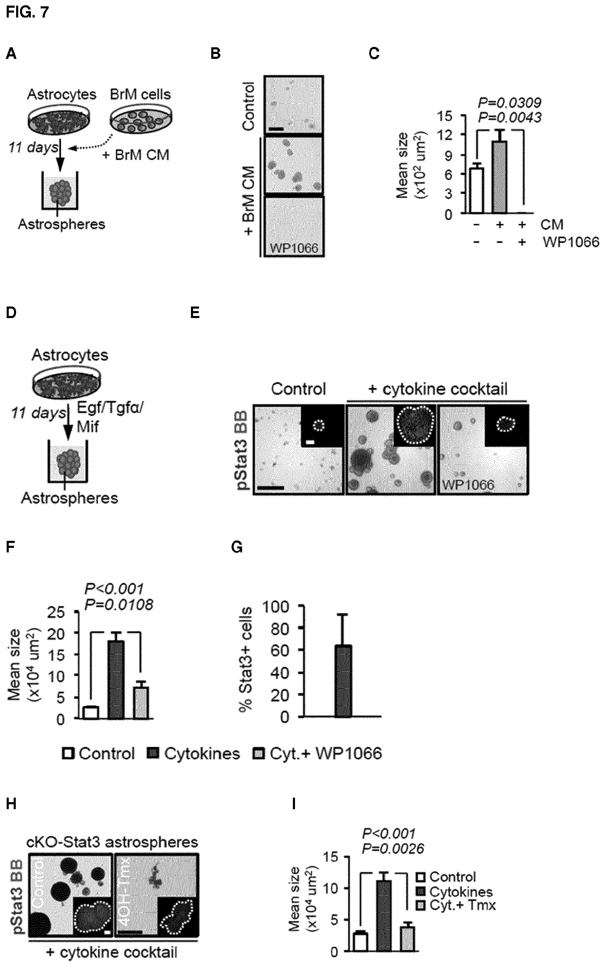

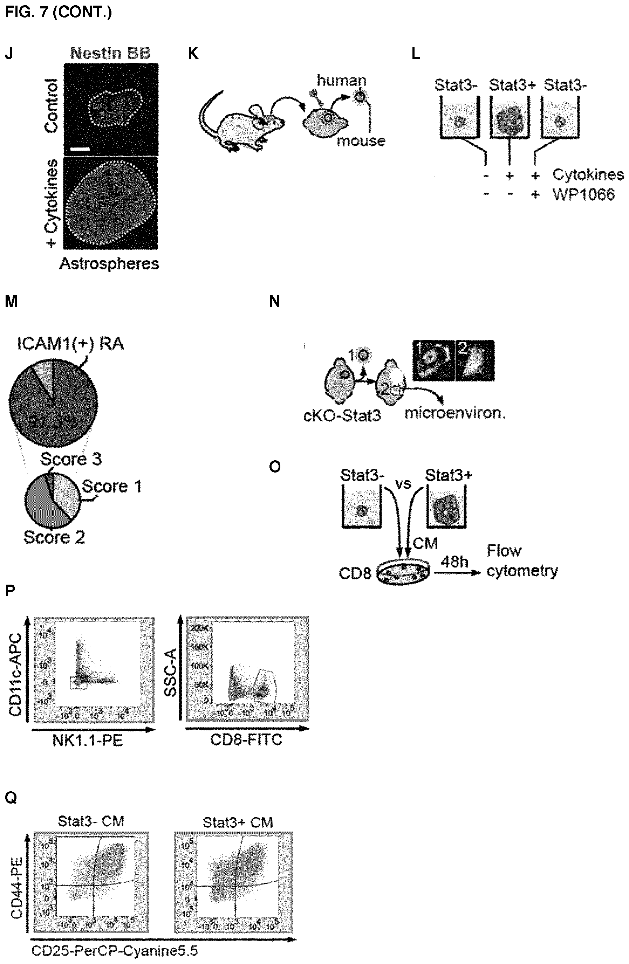

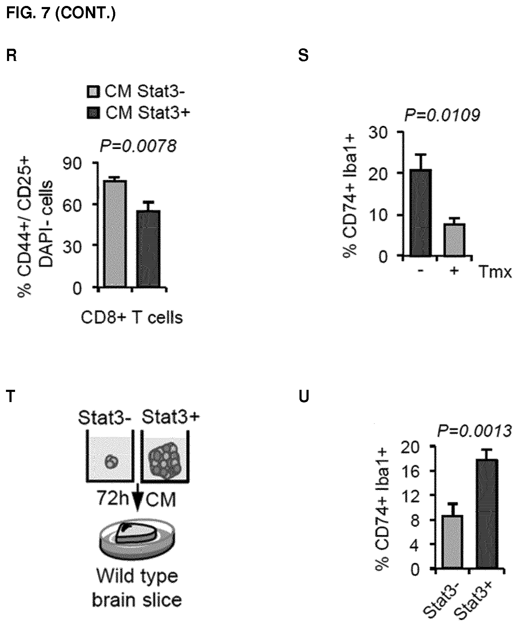

**Figure** 7. De novo induced Stat3 activity confers a differential functional identity to reactive astrocytes. A. Schema of experimental design. The formation of astrospheres was analyzed after incubation of primary astrocytes with conditioned media (CM) from brain metastatic (BrM) cells. B. Representative images of astrospheres incubated with and without BrM CM. More and bigger astrospheres were found when incubated with BrM CM. Scale bar: 1 mm. C. Quantification of the size of astrospheres induced by the CM of H2030-BrM cell line. Error bars, S.E.M. (n=10 wells control, 8 wells CM H2030-BrM, 3 wells H2030- BrM with WP1066). P value is calculated using two-tailed t test. D. Schema of experimental design. A cocktail of three cytokines was used to evaluate their ability to induce astrospheres. E. Representative images showing astrospheres under different experimental conditions. High magnification shows pStat3 (Tyr705) in astrospheres. WP1066 impairs the formation of astrospheres in the presence of the cytokine cocktail. Scale bar: 2 mm, high magnification 100 µm. F. Quantification of the experiment in E. Error bars, S.E.M. (n≥6 wells per condition). P value is calculated using two-tailed t test. G. Quantification of pStat3+ cells in astrospheres from experiment in E. Error bars, S.E.M. (n≥6 wells per experimental condition). H. Representative images of cKO-Stat3 derived astrospheres that were generated in the presence or absence of 4OH-Tamoxifen (1µM) and a cytokine cocktail as in shown in D. Scale bar: 2 mm, high magnification 100 µm. I. Quantification of the experiment in H. Error bars, S.E.M. (n=4 wells per condition). P value is calculated using two-tailed t test. J. Astrospheres generated in the presence of the cytokine cocktail are positive for Nestin. Scale bar: 250 µm. K. Schema of experimental design. Established metastases from human BrM cell lines were dissected along with the surrounding mouse microenvironment to obtain RNA and perform qRT-PCR. L. Schema of experimental design. Astrospheres generated in the presence of a cytokine cocktail (as in D) and the Stat3 inhibitor WP1066 were used to evaluate Stat3 downstream genes. M. Percentage of human brain metastases with ICAM1 positive staining in reactive astrocytes and their immunohistochemistry score. N. Schema of experimental design. Brains from Tamoxifen treated and untreated cKO-Stat3 mice were obtained two weeks after IC injection of B16/F10-BrM cells. Lesions were microdissected *ex vivo* and Luciferase-tissue was processed to perform qRTPCR analysis. O. Schema of experimental design. Flow sorted CD8 T cells obtained from C57BL/6 wild type mice were activated *in vitro* before being incubated with conditioned media (CM) generated by astrospheres. 48 hours after, CD8+ T cells were analyzed by flow cytometry. P. Dot plots showing sorted CD11c-NK1.1-CD8+ cells that were used in these experiments. Q. Representative flow cytometry analysis using pre-activated CD8+ lymphocytes incubated with conditioned media generated by Stat3- and Stat3+ astrospheres. R. Quantification of experiments shown in G. Error bars, S.E.M. (n=8/9 co-cultures per condition from three independent experiments). P value is calculated using two-tailed t test. S. Quantification of Iba1+ and CD74+ cells in images of established metastases. Error bars, S.E.M. (Control: n=11 field of view, from 4 metastases, 2 brains; Tamoxifen: n=8 field of view, from 4 metastases, 3 brains). P value is calculated using two-tailed t test. T. Schema of experimental design. CM: conditioned media from astrospheres. U. Quantification of the experiment in T. Error bars, S.E.M. (Control: n=12 field of view, from 2 organotypic cultures; 4OHTmx: n=14 field of view, from 2 organotypic cultures). P value is calculated using two-tailed t test.

### EXAMPLES

The following examples illustrate the advantages of the inhibition of STAT3 in GFAP expressing cells for reducing brain metastases. The experimental results indicate that brain metastatic cells induce and maintain the co-option of a pro-metastatic program driven by Stat3 in a subpopulation of reactive astrocytes (RA) as part of their adaptation to the brain. Moreover, these results show that the anti-metastatic effects of Stat3 blocking in RA from the brain of mice and humans are independent of primary tumor source and of the prognosis of the disease and are useful for the treatment of brain metastasis.

### Example 1: Stat3 expression in reactive astrocytes

A total of 29 paraffin embedded human brain metastases from lung adenocarcinoma (21 samples), breast adenocarcinoma (6 samples) and melanoma (2 samples) obtained through surgical resection (table 1) were stained for phosphorylated (Tyr705) STAT3 and GFAP and blindly evaluated and scored by clinical neuropathologists. Protein expression was semiquantitatively assessed taking into account the intensity and the extension of the positivity (0: no expression; 1: mild-moderated and focal expression; 2: strong focal expression or moderate diffuse expression; 3: strong and diffuse expression). 96.5% of these samples were positive for phosphorylated STAT3 in reactive astrocytes, of which 60.7% scored above 1. RA with phosphorylated STAT3 (pSTAT3+) were found intermingled with negative ones. Human brain metastases have limited numbers of cancer cells with phosphorylated STAT3. The importance of STAT3 in the progression of the disease became evident when both compartments (cancer cells and RA) were analyzed with respect to survival from diagnosis of primary tumor. High levels of STAT3 (2 or higher) were prognostic of worst outcome only when evaluated in RA (Figs. 1A and B).

Similar to human samples, analysis of experimental brain metastasis with different oncogenomic profiles and from various primary tumor sources (H2030-BrM, PC9-BrM, 482N1, 393N1, MDA231-BrM, Hcc1954-BrM, ErbB2-BrM, B16/F10-BrM from Valiente, M. et al. Cell 156, 1002-1016 (2014); Bos, P. D. et al. Nature 459, 1005-1009 (2009); Nguyen, D. X. et al. Cell 138, 51-62 (2009); Malladi, S. et al. Cell 165, 45-60 (2016) and those disclosed herein for the first time) showed specific activation of Stat3 signaling in the local microenvironment surrounding brain metastasis. Co-localization with GFAP indicated that the main cellular source of active Stat3 is RA. Although the presence of Stat3 in other components of the reactive brain metastasis microenvironment has been previously probed none of them disclose the high levels of active Stat3 detected in RA *in situ.*

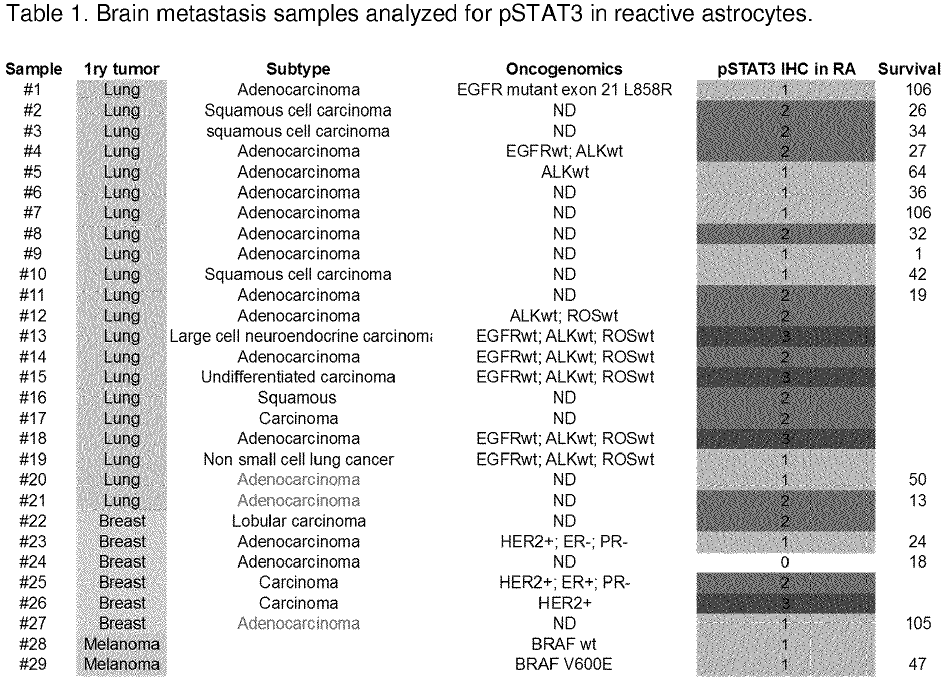

Survival is expressed in months. ND: not determined.

### Example 2: Stat3 inhibition in GFAP expressing cells

To test the functional contribution of pStat3+ RA in brain metastasis we generated *GFAP-Cre*/*ERT2;Stat3loxP*/*loxP* (cKO-Stat3) mice in which syngeneic brain metastatic (BrM) cells, labeled with Luciferase and Gfp (Green fluorescent protein), were intracardiacally inoculated. We used two BrM models including lung adenocarcinoma model (482N1) and melanoma model (B16/F10-BrM). These syngeneic models have gained brain tropism while retaining an aggressive extracranial metastatic potential (Valiente, M. et al. Cell 156, 1002-1016 (2014) and also as disclosed herein). Three days after intracardiac inoculation of B16/F10-BrM in cKO-Stat3, we injected tamoxifen and analysed brain metastasis twelve days later with bioluminescence imaging (BLI). We confirmed Stat3 depletion from RA under these experimental conditions (Fig.2). A significant reduction in brain metastasis burden was detected in cKO-Stat3 treated with tamoxifen (n=29 brains, *P=0.017*) (Figs. 3 A and B), which was also validated with histology (Fig. 3C and D). In order to analyze the effect of Stat3 deletion in RA once brain metastases have developed, and given the limited time available *in vivo* before mice reach the humane endpoint, we cultured *ex vivo* cKO-Stat3 brain slices containing 2 weeks old lesions grown in mice (Fig. 4A). Depletion of Stat3 from RA upon addition of 4OH-Tamoxifen (4OH-TMx) to brain cultures impaired the growth of established metastases from B16/F10-BrM (n≥ 8 brain slices per condition, *P=0.012*) (Fig. 4B) and 482N1 (n≥ 4 brain slices per condition, *P=0.013*) as scored with BLI (Fig. 4C). Established brain metastases decrease cancer cell proliferation and increase apoptosis when Stat3 was depleted from RA (Figs. 4D and E). These results suggest a strong dependency of brain metastasis on a pro-metastatic microenvironment regulated by Stat3+ RA. We also found that the previously reported inhibition of brain metastasis following Stat3 blocking with the JAK-2 inhibitor WP1066 is phenocopied by targeting Stat3 in RA (Figs. 4 D-F) but not by targeting it in cancer cells (Figs. 4F-H).

### Example 3: the anti-metastatic effect of silibinin in brain metastasis is derived from blocking Stat3 activation in RA.

In organotypic cultures silibinin decreased Stat3 activity in the microenvironment surrounding established brain metastasis, which showed lower values of bioluminescence than controls (Fig. 5A-D). Importantly, BrM cell lines used in these experiments were not sensitive to silibinin when treated *in vitro,* discarding any confounding effect of this treatment. To perform the *in vivo* treatment of experimental brain metastasis, we used the silibinin formulation contained in Legasil® to mimic the human situation. Treatment of B16/F10-BrM brain metastasis *in vivo* (Fig. 5E) reproduced *ex vivo* results inducing a decrease in brain metastasis burden, as scored by BLI (Fig. 5F) and histology (Fig. 5G), and impairing Stat3 signaling in the microenvironment. Interestingly there was no difference between treated and untreated animals with respect to extracranial metastases (Fig. 5 H).

### Example 4: clinical study

Legasil® was used in a clinical study with a larger cohort of 18 lung cancer patients with brain metastases.

Patients had different histology and oncogenomic profiles:
Sex: Male 11 (61%), Female 7 (39%)
Age: <70 y 16 (89%), >70 y 2 (11%)
Karnofsky performance status (PS): >70% 8 (44%), ≤70% 10 (56%)
Extracraneal metastases: Present 16 (89%), Absent 2 (11%)
Histology: Small cell 2 (11%), Squamous 1 (5.5%), Adenocarcinoma 14 (78%), Large Cell 1 (5.5%)
Brain Metastases status: Newly diagnosed 13 (72%), Progressive disease 5 (28%)

**Graded prognostic assessment (GPA) prognostic class:**

| | |
|---|---|
| 3.5-4.0 | 0 |
| 2.5-3.0 | 1 (5.5%) |
| 1.5-2.0 | 8 (44.5%) |
| 0.0-1.0 | 9 (50%) |

Gene status: EGFR pos 4 (22%), ALK pos 1 (5%)

Patients also had failed to previous treatments including whole brain radiation therapy (WBRT) and one or more lines of systemic therapy:

**Systemic treatment administered to the patients:**

| **Previous line to brain** | **5 (28%)** |
|---|---|
| **M1** | |
| Carboplatin-vinorelbine & lung radiotherapy (stage III) | 3 |
| Cisplatin-pemetrexed (stage IV) | 1 |
| Gefitinib (stage IV) | 1 |

| **First line** | **18 (100%)** |
|---|---|
| Carboplatin-pemetrexed | 8 |
| Cisplatin-pemetrexed | 1 |
| Pemetrexed | 3 |
| Carboplatin-etoposide | 2 |
| EGFR TKI | 3 |
| Carboplatin-gemcitabine | 1 |

| **Second line** | **14 (78%)** |
|---|---|
| Carboplatin-pemetrexed | 3 |
| Pemetrexed | 2 |
| Docetaxel | 3 |
| Docetaxel-nintedanib | 1 |
| Nivolumab | 1 |
| Carboplatin-gemcitabine | 1 |
| Carboplatin-etoposide | 1 |
| Crizotinib | 1 |
| Erlotinib | 1 |

| **Third line** | **6 (33%)** |
|---|---|
| Carboplatin-gemcitabine | 1 |
| Erlotinib | 1 |
| Docetaxel-nintedanib | 1 |
| 3rd generation EGFR TKI* | 1 |
| 3rd generation ALK TKI* | 1 |
| Nivolumab | 1 |

| **Fourth line** | **2 (11%)** |
|---|---|
| Pemetrexed | 1 |
| Erlotinib | 1 |

| | |
|---|---|
| * Treatment received in a clinical trial, the patient stopped silibinin supplementation before inclusion to the clinical trial and during all the treatment with this experimental drug according to study protocol. | |

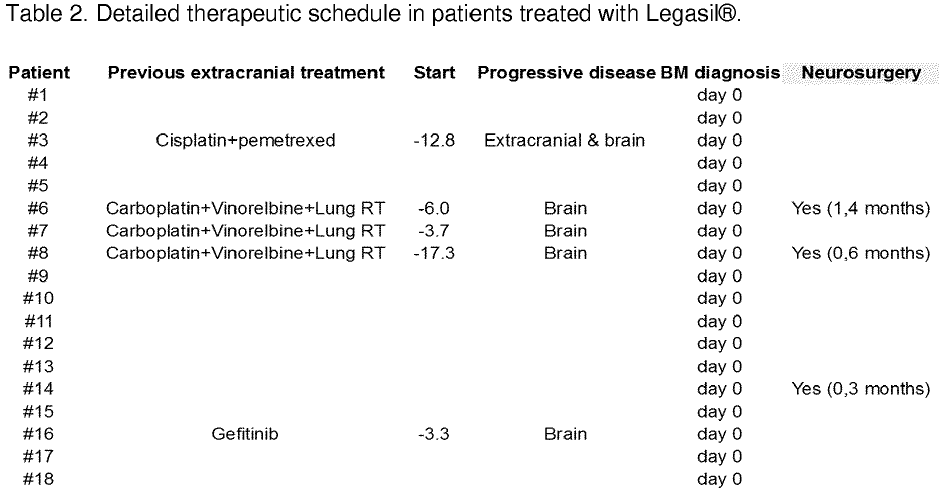

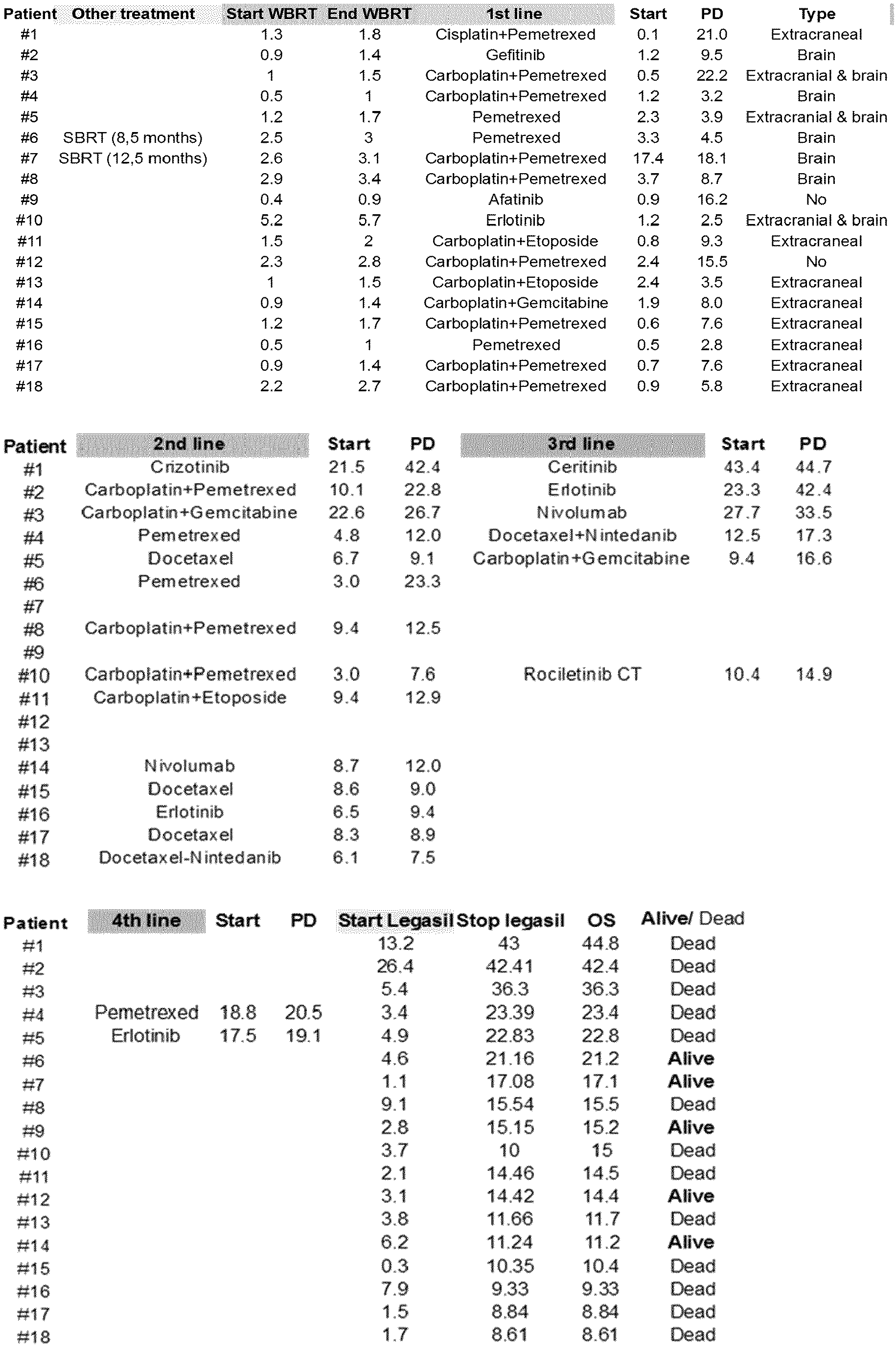

Initiation of Legasil® therapy as a single agent (3 patients under palliative care) or in combination with additional chemotherapy (15 patients) induced an overall response rate (ORR) in the brain of 75% (Fig. 6A), including 3 complete responses (20%) and 10 partial responses (55%). The three patients who started to take Legasil® under palliative care received additional lines of chemotherapy when their general status improved and CNS responses were confirmed by MRI. Legasil® in combination with different lines of chemotherapy was not as effective controlling extracranial disease (primary tumor and metastases out of the brain) (ORR extracranially 40%; ORR intracraneal vs extracranial *P=0.0079*) (Fig. 6B). In fact most of the patients ended up progressing outside the CNS. Six patients showed local progression in the brain while being treated with Legasil®, with four of them progressing with new single or multiple brain micro-metastasis that remained below 1 cm and did not generate edema. At data cutoff, 5 (27.8%) patients remained alive (12.1-22 months after being diagnosed with brain metastases). Overall survival (OS) from diagnosis of brain metastasis was significantly superior compared with expected OS calculated by Lung-molGPA (brain GPA Index)33 (median Legasil®: 22.8 months [95% Cl 10.7-35] vs Controls: 6.9 months [4.2-9.6]; *P=0.001*) (Fig. 6C).

### Example 5: De novo induced Stat3 activity confers a differential functional identity to reactive astrocytes.

Given the absence of Stat3 signaling in GFAP+ reactive astrocytes surrounding brain metastasis initiating cells at early stages of colonization, but their abundance later on surrounding well developed lesions, we hypothesized that Stat3 would become activated once important damage and cytokine concentrations had been produced by the growth of metastatic lesions. To test this hypothesis *in vitro* we added conditioned media (CM) from various BrM cell lines to primary astrocytes (Fig. 7A). Although we did not detect major changes in astrocytes cultured under standard *in vitro* conditions, significant differences emerged when astrocytes were plated in conditions that favour sphere formation. It was previously shown that the ability to generate spheres was not exclusive of neural stem cells but that mature astrocytes could do it in the absence of tumor suppressors and after stimulation with specific cytokines (Bachoo, R. M. et al. Cancer Cell 1, 269-277 (2002)), which might reflect their ability to de-differentiate and acquire additional capabilities. In this sense pStat3+ RA have been recently shown to provide regenerative potential after acute injury (Anderson, M. A. et al. Nature 532, 195-200 (2016)), probing that *in vivo* this subpopulation could have different functions than pStat3- RA. We found that Stat3 activating cytokines were frequently upregulated in a broad collection of BrM cancer cell lines, in which three of them (Egf, Tgfα and Mif) were ubiquitously represented. Astrocytes incubated with CM from BrM cells formed more numerous and bigger astrospheres in a Stat3 dependent manner (Fig. 7B and C). This phenotype was also found by incubating primary astrocytes with a cytokine cocktail including Egf, Tgfα and Mif (Fig. 7D to G). In a series of complementary experiments, we observed that cKO-Stat3 derived astrocytes are unable to generate astrospheres when induced by the cytokine cocktail in the presence of 4OHtamoxifen (Fig. 7H and I). Interestingly we also found that the neural stem cell marker Nestin is specifically enriched in pStat3+ astrospheres (Fig. 7 J) as well as in pStat3+ RA associated with brain metastasis, which suggests that pStat3+ RA are less differentiated than pStat3- RA.

To investigate the genetic program downstream of Stat3 in brain metastasis associated RA we analyzed Stat3 target gene expression (Anderson, M. A. et al. Nature 532, 195-200 (2016)) in different experimental models. We confirmed the presence of Stat3 induced genes (Icam1, Ctgf, Timp1, Pdl1, Ptx3, Lcn2, Dnmt3, Vegfa, Tgfβ, Mif) in the reactive microenvironment of brain metastasis xenografts from three different human cancer cells (Fig. 7K and L), some of which were also validated *in situ* in reactive astrocytes of mice and human patients with brain metastasis (Fig. 7M). By triple immunostaining, we found that Icam1 co-localizes with pStat3 (Tyr705) (r) and Ctgf with GFAP Immunohistochemistry of ICAM1 in human brain metastases samples showed that ICAM1 was found in the majority of samples in reactive astrocytes (Fig. 7 M). High magnification showed ICAM1+ cell morphology compatible with a reactive astrocyte in the vicinity of cancer cells.

We also found that these genes are also enriched in Stat3+ astrospheres (Fig. 7L), and decreased in cKO-Stat3 brains after the activation of Cre recombinase (Fig. 7N). For the analysis ten brains per condition were analysed and control values were obtained from the use of five cKO-Stat3 brains from mice not injected with BrM cells.

In patients, brain metastases are infiltrated with lymphocytes (Berghoff, A. S. et al. Oncoimmunology 5, e1057388 (2016)). and experimental models recapitulate this feature. We hypothesized that, given the well-established role of Stat3 as an immune regulatory molecule (Jones, L. M. et al. Cancer Res 76, 1416-1428 (2016); Wang, T. et al. Nat Med 10, 48-54 (2004)), and the link between regenerative potential and immunosuppression (Drukker, M. & Benvenisty, N. Trends Biotechnol 22, 136-141 (2004); Aurora, A. B. & Olson, E. N. Cell Stem Cell 15, 14-25 (2014); Dubeykovskaya, Z. et al. Nat Commun 7, 10517 (2016)), pro-metastatic functions of pStat3+ RA could include the blockade of local anti-tumor activities. We tested this hypothesis incubating activated CD8+ T cells (Cho, J.-H. et al. J Immunol 191, 5559-5573 (2013))with conditioned media from pStat3+ astrospheres (Fig. 7O). This experiment showed that the secretome of pStat3+ RA is sufficient to decrease the percentage of lymphocytes co-expressing T cell activation surface markers CD25 and CD44 (Rosenblum, M. D., et al. Nat Rev Immunol 16, 90-101 (2016)) (Fig. 7P to R). Since targeting Stat3 also impairs brain metastasis viability in T cell depleted animals (Lee, H.-T. et al. Oncotarget 6, 10016-10029 (2015)) (Fig. 4H, Fig. 5 C) additional pro-metastatic roles of pStat3+ RA might exist. In contrast to RA, microglia/ macrophages, although enriched at the margins of the lesion, are also found at the lesion core intermingled with highly packed cancer cells (Valiente, M. et al. Cell 156, 1002-1016 (2014).Sevenich, L. et al. Nat Cell Biol 16, 876-888 (2014)). We found that microglia/ macrophages, labeled with Iba1+, that infiltrate the lesion are enriched in CD74. CD74+ microglia/ macrophages have been shown to promote tumor growth blocking innate anti-tumor responses (Ghoochani, A. et al. Oncogene 35, 6246-6261 (2016)). We observed that cKO-Stat3 brains treated with tamoxifen have less CD74+/lba1+ cells infiltrating metastasis (Fig. 7S). In addition, we evaluated the effect of pStat3+ astrospheres on CD74+/Iba1+ cells by incubating wild type brain slices with the CM from astrospheres during 3 days (Fig. 7T). Our results show that the CD74+/lba1+ microglia/ macrophage population increases only in the presence of CM derived from pStat3+ astrospheres (Fig. 7U). Thus, the pro-metastatic role of Stat3+ RA may involve its ability to influence various components of the immune system.

### Example 6: methods

### Animal studies

All animal experiments were performed in accordance with a protocol approved by the CNIO, Instituto de Salud Carlos III and Comunidad de Madrid Institutional Animal Care and Use Committee. Athymic nu/nu (Harlan), and C57BL/6 mice 4-6 weeks of age were used. cKO-Stat3 was generated by breeding GFAP-CRE/ERT2 (B6.Cg- Tg(GFAP-cre/ERT2)505Fmv/J, Jackson Labs, ref. 012849) with Stat3loxP/loxP16.

Brain metastatic derivative of a syngeneic B16/F10 model (B16/F10-BrM) was established according to a previous protocol (Bos, P. D. et al. Nature 459, 1005-1009 (2009); Nguyen, D. X. et al. Cell 138, 51-62 (2009)). Brain colonization assays were performed injecting 100 µl of PBS into the left ventricle containing 100,000 cancer cells. Brain colonization was analyzed in vivo and ex vivo by bioluminescence imaging. Anesthetized mice (Isofluorane) were injected retro-orbitally with D-Luciferin (150 mg/kg) and imaged with an IVIS Spectrum Xenogen machine (Caliper Life Sciences). Bioluminescence analysis was performed using Living Image software, version 3. Ex vivo values at the endpoint were normalized to the BLI values of the head in vivo before starting treating with tamoxifen (l.p., 1 mg/day) three days after injection of the cancer cells. Tamoxifen was administered until the end of the experiment. Silibinin in the formula of Legasil® was administered by oral gavage daily the same day cancer cells were inoculated (200mg/kg) and until mice reached the endpoint of the experiment.

### Brain slice assays

Organotypic slice cultures from adult mouse brain were prepared as previously described (Valiente, M. et al. Cell 156, 1002-1016 (2014)). Organotypic cultures also included brains obtained at the endpoint of metastatic disease (5-7 weeks in human brain metastatic models, 2 weeks in mouse brain metastatic models), when brain lesions are established. In brief brains were dissected in Hank's balanced salt solution (HBSS) supplemented with HEPES (pH 7.4, 2.5 mM), D-glucose (30 mM), CaCl₂ (1 mM), MgSO₄ (1 mM), NaHCO₃ (4 mM), and embedded in low-melting agarose (Lonza) preheated at 42° C. The embedded brains were cut into 250 µm slices using a vibratome (Leica). Slices were divided at the hemisphere into two pieces. Brain slices were placed with flat spatulas on top of 0.8 µm pore membranes (Millipore) floating on slice culture media (Dulbecco's modified Eagle's medium [DMEM], supplemented HBSS, fetal bovine serum 5%, L-glutamine (1 mM), 100 IU/ml penicillin, 100 mg/ml streptomycin). Brain slices were imaged to confirm the presence of established metastases using BLI. If brain slices receive a treatment involving Tmx, or were compared to a condition in which Tmx was present, BLI was acquired 12h after plating (Day 0) and 4 days after the addition of the inhibitor (Day 5). Growth rate was obtained by comparing fold increases between day 5 and day 0.

Treatments to brain slices were given once during the experiment (4OH-Tmx, 1 µM, Sigma-Aldrich, ref. H7904; WP1066, 10 µM, Merck, ref. 573097; Doxycycline, 1 µg/ml, Sigma-Aldrich, ref. D9891; silibinin, 100 µM, Sigma-Aldrich, ref. S0417) by adding them to the media. If inhibitors were added to wild type mice where recombination was not necessary, slices were incubated 3 days. If slices were obtained from brains without metastases 3x10⁴ cancer cells suspended in 2 µl of culture media were placed on the surface of the slice and incubated for 3 days. BrdU pulse (0.2 mg/ml, Sigma-Aldrich, ref. B9285) was given by adding it in the media four hours before fixation. Brain slices were fixed in paraformaldehyde (4%) overnight, and then free-floating immunofluorescence was performed. Slices were mounted with Mowiol-Dabco anti-fade reagent. Analysis of the knockdown level in H2030-BrM (Fig. 4F) was analyzed in a homogenate containing the mouse brain tissue and human cancer cells. Use of human primers allowed evaluating *STAT3* levels in cancer cells.

### Cell culture

Human and mouse brain metastatic cell lines were previously described (Valiente, M. et al. Cell 156, 1002-1016 (2014); Bos, P. D. et al. Nature 459, 1005-1009 (2009); Nguyen, D. X. et al. Cell 138, 51-62 (2009); Malladi, S. et al. Cell 165, 45-60 (2016)). B16/F10-P cells were injected intracardiacally to obtain brain metastatic derivatives. Briefly, a cell suspension containing 10⁵ B16/F10-P cells expressing a Luciferase construct (Addgene ref. 19166, Blasticidine resistance)37, in a volume of 100 µl was injected in the left cardiac ventricle of anesthetized 4-6 week-old C57BL/6 mice. Tumor development was monitored by weekly bioluminescence imaging using the IVIS-200 imaging system. Brain lesions were localized by *ex vivo* bioluminescence imaging, and resected under sterile conditions. Tissue was minced and placed in culture medium containing a 1:1 mixture of DMEM/Ham's F12 supplemented with 0.125% collagenase III and 0.1% hyaluronidase. Samples were incubated at 37° C for 1 h, with gentle rocking. After collagenase treatment, cells were briefly centrifuged, resuspended in 0.25% trypsin, and incubated at 37° C for 15 min. Cells were resuspended in culture media and allowed to grow to confluence on a 10 cm dish. Two additional rounds of *in vivo* selection were performed. Brain metastatic cells (BrM3) were fluorescently labelled with a lentiviral vector encoding ZsGFP and sorted for further propagation in culture or inoculation in mice.

MDA231-BrM2 (abbreviated as MDA231-BrM), ErbB2-BrM2 (abbreviated as ErbB2- BrM), 373N1, 393N1, 482N1, 2691N1, B16/F10-BrM3 (abbreviated as B16/F10-BrM) where cultured in DMEM media supplemented with 10% fetal bovine serum (FBS), 2 mM L-Glutamine, 100IU/ml penicillin/streptomycin and 1 mg/ml amphotericin B. H2030-BrM3 (abbreviated as H2030-BrM), PC9-BrM3 (abbreviated as PC9-BrM) and HCC1954-BrM1 (abbreviated as Hcc1954-BrM) were cultured in RPMI1640 media supplemented with 10% fetal bovine serum (FBS), 2 mM L-Glutamine, 100IU/ml penicillin/streptomycin, and 1 mg/ml amphotericin B. For lentivirus production, 293T cells were cultured in DMEM media supplemented with 10% fetal bovine serum (FBS), 2 mM L-Glutamine, 100 IU/ml penicillin/streptomycin, and 1 mg/ml amphotericin B. Mouse astrocytes were obtained from one to three day old pups. In brief, brains were mechanically dissociated, filtered through 100 mm filters and cell suspension cultured in a petri dish during the next 7 days. On day 7, the dish was incubated overnight at 37° C with gentle shaking. Next day media was changed and astrocyte enrichment confirmed with > 90% of cells staining positive for GFAP.

### T cells in vitro experiments

T cells were obtained from the spleen of 10-15 week-old C57BL/6 mice. The whole organ was pressed through a 70 µm cell strainer and red blood cells were lysed with ACK Lysing Buffer (Lonza, ref. 10-548E). Cells were resuspended in BSA 0.1% in PBS and incubated with FC-Block (BD, ref. 553141). CD11c- NK1.1- CD8+ cells were isolated using BD FACSAria Ilu cell sorter (BD, San Jose CA) and the following conjugated antibodies: anti-mouse CD8a-FITC (1:200; Tonbo Biosciences ref.35- 008-1), anti-mouse NK1.1-PE (1:200; e-Bioscience ref. 12-5941-82) and anti-mouse CD11C-APC (1:200; BD Biosciences ref. 550261). Then, CD8+ cells were activated with anti-mouse CD3e clone 145-2C11 (1µg/ml, BD Biosciences ref. 553066) coated plates, soluble anti-mouse CD28 (37.51) (1µg/ml; Tonbo Biosciences ref. 70-0281- U500) and mouse IL2 (1µg/ml; Miltenyi Biotec ref. 130-094-054) in RPMI medium supplemented with 10% FBS and Penicillin-Streptomycin during one day before conditioned medium (CM) from Stat3+ and Stat3-astrospheres was added. Two days after addition of CM, flow cytometry (BD FACSCanto II) was performed using CD44-PE (1:200; BD Biosciences ref. 553134) and CD25-PerCP-Cyanine5.5 (1:200; e-Bioscience ref. 45-0251-80). Pulse processing and DAPI were used to exclude cell aggregates and dead cells. Between 2,500 and 20,000 single live events were acquired and all data was analyzed using FlowJo v10.0 (Treestar, OR).

### qRT-PCR

Whole RNA was isolated using RNAeasy Mini Kit (QIAGEN). 1000 ng RNA was used to generate cDNA using iScript cDNA Synthesis Kit (Bio-Rad, ref. 1708890). RNA obtained from mouse brains included microdissected established metastases from human BrM cells, in which the microenvironment was analyzed by using mouse primers, and microdissected BrM metastases that had grown in cKO-Stat3 brains treated or not with Tamoxifen (Tmx). In this case the microenvironment was separated by dissecting Luciferase- tissue immediately adjacent to Luciferase+ cancer cells. Microdissection in cKO-Stat3 was initially validated by confirming the absence of Gfp+ cells using flow cytometry (data not shown). RNA from BrM cell lines was obtained from a confluent well from a 6 well plate. RNA from astrospheres was obtained from cells cultured in 24 well plates or 10 cm plates, both low attachment. Gene expression was analyzed using SYBR green gene expression assays (GoTaq® qPCR Master Mix Promega, ref. A6002).

Primers used for human genes (5'->3', forward;reverse):
- STAT3 (CTGAGCTGGCAGTTCTCCTC (SEQ ID NO: 2);
   GAAGGTGCCTGGAGGCTTAG (SEQ ID NO: 3)).

Primers used for mouse genes (5'->3', forward ;reverse):
- Stat3 (CAGAAAGTGTCCTACAAGGGCG (SEQ ID NO: 4);
   CGTTGTTAGACTCCTCCATGTTC (SEQ ID NO: 5)),
- GFAP (ACGTTAAGCTAGCCCTGGAC (SEQ ID NO: 6);
   GGTGAGCCTGTATTGGGACAA (SEQ ID NO: 7)),
- Icam1 (TTTGAGCTGAGCGAGATCGG (SEQ ID NO: 8);
   AGAGGTCTCAGCTCCACACT (SEQ ID NO: 9)),
- Ctgf (GTGCCAGAACGCACACTG (SEQ ID NO: 10);
   CCCCGGTTACACTCCAAA (SEQ ID NO: 11)),
- Timp1 (GAGACACACCAGAGCAGATACC (SEQ ID NO: 12);
   TGGTCTCGTTGATTTCTGGGG (SEQ ID NO: 13)),
- Pdl1 (TTCACAGCCTGCTGTCACTT (SEQ ID NO: 14);
   CTCTCCCCCTGAAGTTGCTG (SEQ ID NO: 15)),
- Lcn2 (GAACTTGATCCCTGCCCCAT (SEQ ID NO: 16);
   TTCTGATCCAGTAGCGACAGC (SEQ ID NO: 17)),
- Ptx3 (CTTCCCAATGCGTTCGAAGAAGATTTTTGG (SEQ ID NO: 18);
   AACACTTAAGAAACATACTGGGCTCCTCCG (SEQ ID NO: 19)),
- Dnmt3a (GCCGAATTGTGTCTTGGTGGATGACA (SEQ ID NO: 20);
   CCTGGTGGAATGCACTGCAGAAGGA (SEQ ID NO: 21)),
- Vegfa (CCTGGCCCTCAAGTACACCTT (SEQ ID NO: 22);
   TCCGTACGACGCATTTCTAG (SEQ ID NO: 23)),
- Tgfβ1 (CTGCTGACCCCCACTGATAC (SEQ ID NO: 24);
   GTGAGCGCTGAATCGAAAGC (SEQ ID NO: 25)),
- Mif (CTTTGTACCGTCCTCCGGTC (SEQ ID NO: 26);
   CGTTCGTGCCGCTAAAAGTC (SEQ ID NO: 27)).

Relative gene expression was normalized to the "housekeeping":
Human genes (5'->3', forward;reverse):
   - B2M (AGATGAGTATGCCTGCCGTG (SEQ ID NO: 28);
      TCATCCAATCCAAATGCGGC (SEQ ID NO: 29)).
Mouse genes (5'->3', forward;reverse):
   - Actin (GGCACCACACCTTCTACAATG (SEQ ID NO: 30);
      GTGGTGGTGAAGCTGTAGCC-3') (SEQ ID NO: 31).

Quantitative PCR reaction was performed on QuantStudio 6 Flex Real-Time PCR System (Applied Biosystems) and analysed using the software QuantStudio 6 and 7 Flex Software.

### Clinical samples and immunohistochemistry

Twenty-nine cases from lung cancer (21 cases), breast cancer (6 cases) and melanoma (2 cases) brain metastasis were obtained from the Department of Pathology, Vall d'Hebron Hospital, the Department of Pathology, Hospital 12 de Octubre and University Hospital of Turin Institutional Review Board in compliance with protocols approved by their respective Institutional Review Board (IRB). Cases were selected by two neuropathologists (E.M.S., A.H.L.) and included cerebral tissue in order to evaluate the microenvironment surrounding brain metastasis. Immunohistochemistry for GFAP (Ventana Medical Biosystem, ref. 760-4345), STAT3 (Tyr705) (Cell Signaling, ref. 9145, 1:100), ICAM1 (Sigma-Aldrich, ref. HPA004877, 1:500), were performed by the Vall d'Hebron Hospital using standardized automated protocols. Immunostains were blindly evaluated and scored by clinical neuropathologists (E.M.S., S.R.C). Expression in astrocytes and metastatic cells was independently evaluated. 17 samples had annotated clinical history related to time to death from diagnosis of the primary tumor.

### Immunofluorescence and immunohistochemistry

Tissue for immunofluorescence was obtained after overnight fixation with PFA 4% at 4 C. Slicing of the brain was done by using a vibratome (Leica) or sliding microtome (Fisher). Both types of brain slices (250 µm and 80 µm respectively) were blocked in NGS 10%, BSA 2%, Triton 0.25% in PBS for 2 hr at room temperature (RT). Primary antibodies were incubated overnight at 4C in the blocking solution and the following day for 30 min at RT. After extensive washing in PBS-Triton 0.25%, the secondary antibody was added in the blocking solution and incubated for 2 hr. After extensive washing in PBS-Triton 0.25%, nuclei were stained with Bis-Benzamide (1 mg/ml; Sigma) for 7 min at RT. Primary antibodies: GFP (Aves Labs, ref. GFP-1020, 1:1,000), pStat3 (Tyr705) (Cell Signaling, ref. 9145, 1:100), GFAP (Millipore, ref. MAB360, 1:1000), Ibal (Wako, ref. 019-19741, 1:500), Nestin (BD Bioscience, ref. 556309, 1:100), NeuN (Millipore, ref. MAB377, 1:500), cleaved caspase-3 (1:500, ref. 9661; Cell Signaling), BrdU (Abcam, ref.ab6326, 1:500), CD8 (Novus Biologicals, ref. NB200-578, 1:100), CD74 (BD Biosciences, ref. 555318, 1:100), CD31 (BD Pharmingen, ref. 550274, 1:100), CD68 (KP1) (Abcam, ref. ab955 1:200). Secondary antibodies: Alexa-Fluor anti-chicken488, anti-rabbit555, anti-mouse555, antimouse633, anti-mouse647 (Invitrogen, dilution 1:300). Same protocol was applied to stain astrospheres. Brain slices or astrospheres were pre-treated with Methanol 10 minutes at -20 C previous to start the immunofluorescence against phosphorylated Stat3. Immunohistochemistry against pStat3 (Tyr705) (Cell Signaling, ref. 9145, 1:100) was performed in paraffin embedded brain sections (5 µm) using standardized automated protocols at the CNIO Histopathology Core Facility.

### Quantification and statistics

Data are represented as the mean values ± S.E.M. When comparisons were done between two experimental groups, an unpaired Student's t test was used. Overall survival was calculated by Lung-molGPA tool and Kaplan-Meier survival curves were generated. For survival curves P values were obtained with log rank (Mantel- Cox) test. Heatmaps were generated using Morpheus software (https://software.broadinstitute.org/morpheus/). For comparatives in cKO-Stat3 and astrospheres a relative scale is used for the representation that takes the minimum and maximum values for each gen. For comparatives in human BrM metastases and 9 BrM cytokines we globally set the minimum as 0 and maximum as 3 and -1.5 and 1.5, respectively, for all the genes.

### Astrosphere assays

Mouse astrocytes were treated with conditioned media from BrM cell lines or with recombinant mouse protein including Egf (0.01 µg/ml, R&D Systems, ref. 2028-EG- 200), Mif (0.1 µg/ml, R&D Systems, ref. 1978-MF-0257CF) and Tgfα (0.1 µg/ml, R&D Systems, ref. 239-A-100) during 96 hours. After this treatment 10⁴ astrocytes were seeded in low attachment plates and incubated for seven days in the presence of the same media to evaluate the ability to form astrospheres. Conditioned media from BrM cell lines were obtained after 3 days incubation of cancer cells in DMEM supplemented with 0.25 % FBS, 2 mM L-Glutamine, 100 IU/ml penicillin/streptomycin and 1 mg/ml amphotericin B. After being collected, the media was filtered and added to astrocytes. Conditioned media from astrospheres was collected, filtered and added to activated CD8 cells (Fig. 7P) or wild type brain slices (Fig. 7T).

### Human patients treated with Legasil®

18 individuals with lung cancer and brain metastasis have been treated with Legasil® in the Catalan Institute of Oncology, Hospital Universitari Dr. Josep Trueta of Girona (Spain). Each capsule of Legasil® contains 210 mg of Eurosil85 (60% of silibinin isoforms). Legasil® (Meda Pharma, Rottapharm-Madaus, Barcelona, Spain) was started at a dose of 2 capsules/day (1-0-1) for 3 days and increased progressively every 3 days to reach up to 5 capsules dosage (1,050 mg of Eurosil85) or until toxicity was observed. Only toxicity observed was diarrhea that remitted upon interruption of the treatment. Once diarrhea was controlled, treatment with Legasil® was reinitiated. Legasil® is available in ambulatory pharmacies of Spain without medical prescription as it is considered a nutritional supplement. All individuals gave written informed consent. The experimental protocol was approved by the Ethics Committee of the Hospital Universitari Dr. Josep Trueta. We certify that all applicable institutional regulations concerning the ethical use of information from human patients were followed during this research. For evaluating the response of brain metastases, one-dimensional measurement has been used following the Response Assessment in Neuro-Oncology (RANO) Brain Metastasis criteria39. Measurable disease was considered when lesions had at least one dimension with a minimum size of 10 mm. Most of patients have been evaluated with brain MRI, with only one patient controlled with brain CT scan. Extracranial response was determined following Response Evaluation Criteria in Solid Tumors (RECIST) criteria version 1.140.

### Image acquisition and analysis

Images were acquired with a Leica SP5 up-right confocal microscope 10X, 20X, 40X and 63X objectives and analyzed with ImageJ software. Astrospheres were imaged with Leica DMi1 inverted microscope.

## Claims

1. Method for the prevention and/or treatment of brain metastasis comprising the administration of a therapeutically effective amount of an inhibitor of the expression or the activation of STAT3 in a patient in need thereof, wherein said inhibitor, in the brain, targets astrocytes and/or GFAP+ cells specifically.

2. The method according to claim 1, wherein the inhibitor comprises an inhibitory anti-STAT3 antibody or an antigen-binding fragment thereof, a decoy peptide, a nucleotide sequence or a STAT3 inhibitor compound.

3. Wherein the nucleotide sequence is or codifies for a gRNA, a siRNA, a microRNA or a shRNA.

4. The method according to any one of the preceding claims, wherein the inhibitor is a nucleotide sequence expressed under the glial fibrillary acidic protein promoter, the glutamate aspartate transporter promoter or the S100 calcium-binding protein B promoter.

5. The method according to any one of the preceding claims, wherein the inhibitor is delivered by a viral vector with lyssavirus rabies virus and/or Mokola virus type envelopes.

6. The method according to any one of claims 1 to 4, wherein the inhibitor is delivered by adeno-associated viral vector, preferably of serotypes 2 or 5.

7. The method according to any one of claims 1 to 4, wherein the inhibitor is delivered in nanoparticles.

8. The method according to any one of claims 1 or 2 or 7, wherein the STAT3 inhibitor compound comprises silibinin, WP1066, WP1220 or honokiol.

9. The method according to any one of claims 1,2 or 7, wherein the STAT3 inhibitor compound is bound to a compound that is preferentially consumed by astrocytes or to an antibody against an antigen preferentially present in astrocytes or an antigen-binding fragment thereof.

10. The method according to the preceding claim, wherein the antigen preferentially present in astrocytes is CD54 or CD274.

11. The method according to any one of the preceding claims, wherein the brain metastasis is a lung cancer, breast cancer, colon cancer, melanoma, renal carcinoma, head and neck cancer, prostate cancer, lymphoma, leukemia, hepatocellular carcinoma, bladder cancer, pancreatic cancer, gastric cancer, esophageal cancer, ovarian cancer or endometrial carcinoma brain metastasis.

12. The method according to any one of the preceding claims, wherein the brain metastasis is a lung cancer, breast cancer, colon cancer, melanoma or renal carcinoma brain metastasis.

13. The method according to any one of the preceding claims, wherein the brain metastasis is a lung cancer, breast cancer or melanoma brain metastasis.

14. The method according to any one of the preceding claims, further comprising administering at least one cytotoxic chemotherapeutic agent, immunotherapy, targeted therapy, surgery, radiotherapy, stereotactic radiosurgery or combinations thereof.

15. The method according to any one of the preceding claims, wherein the patient is human.
